# EUROPEAN PATENT APPLICATION

(11) **EP 3 588 063 A1**
(43) Date of publication of application: **01.01.2020**
(21) Application number: 18191607.3
(22) Date of filing: 30.08.2018
(51) Int. Cl.: G01N 21/64, A61B 5/1459, G01N 33/50, G01J 3/44, A61B 5/00, A61K 49/00, G01N 33/543

(54) **METHOD AND COMPUTER PROGRAM FOR DETERMINING A PHARMACOKINETIC PARAMETER IN A BIOLOGICAL SAMPLE BY MEANS OF FLUORSECENCE CORRELATION SPECTROSCOPY**

(30) Priority: 21.06.2018 EP 18178942
(71) Applicant: Universität Basel, 4001 Basel (CH)
(72) Inventor: Witzigmann, Dominik, V6K 3X8, Vancouver BC (CA); Einfalt, Tomaz, 4054 Basel (CH); Sieber, Sandro, 4052 Basel (CH); Palivan, Cornelia, 2503 Biel/Bienne (CH); Huwyler, Jörg, 4144 Arlesheim (CH)
(74) Representative: Schulz Junghans Patentanwälte PartGmbB

(57) **Abstract**

The invention relates to a fluorescence correlation spectroscopy method for determination of a pharmacokinetic parameter in a biological sample comprising at least the steps of:
- Providing (100) a biological sample (1) comprising a luminescent agent (2);
- Exciting (200) the sample with light such that the luminescent agent is excited and emits photons;
- Recording (300) the emitted photons;
- Calculating (400)a plurality of correlation functions from the recorded photons, wherein the correlation functions are calculated for temporally subsequent or overlapping record portions comprising a plurality of consecutively recorded photons;
- Determining (500) for each correlation function at least one characteristic parameter (4) from the correlation function and determining a temporal course (3) of the at least one characteristic parameter (4);
- From the temporal course (3) of the at least one estimated characteristic (4) parameter determining (600) at least one pharmacokinetic parameter;
- Displaying (700) the at least one pharmacokinetic parameter.

## Description

The invention relates to a method and a computer program for the determination of a pharmacokinetic parameter in a biological sample by means of fluorescence correlation spectroscopy.

Pharmacokinetic properties of test compounds are currently investigated in rodents using radioactively labeled agents. From a regulatory and experimental perspective this is challenging.

From the measured radioactivity, and particularly from the temporal course of the radioactivity and the distribution of the radioactively labeled agent in the organs and tissue samples obtained from the rodent, pharmacokinetic parameters such as the area under the curve are determined.

However, radioactive labeling is elaborate, does not give structural information and requires specific precautions in the laboratory. In addition, determination of pharmacokinetic parameters using common methods can only be performed *ex vivo* and therefore requires an additional sampling and sample preparation step which is labor intensive and a potential source of experimental errors.

The object of the present invention is to overcome the drawbacks associated with radioactive labelling.

The problem is solved by a method according to claim 1 as well as a computer program according to claim 15. Advantageous embodiments are listed in the subclaims.

According to claim 1 a fluorescence correlation spectroscopy method for determination of a pharmacokinetic parameter in a biological sample comprises at least the steps of:
- Providing a biological sample comprising a luminescent agent;
- particularly arranging and positioning the sample in a fluorescence measurement system configured to perform fluorescence correlation spectroscopy measurements;
- Exciting the sample with light, particularly from a light source of the fluorescence measurement system such that the luminescent agent is excited and emits photons;
- Recording the emitted photons;
- Calculating a plurality of correlation functions particularly for a plurality of time lags from the recorded photons, wherein the correlation functions are calculated for and from temporally subsequent or overlapping record portions comprising a plurality of consecutively recorded photons;
- Determining for each correlation function at least one characteristic parameter from the correlation function such that a temporal course of the at least one characteristic parameter is determined;
- From the temporal course of the at least one determined characteristic parameter determining at least one pharmacokinetic parameter particularly associated with the sample and the luminescent agent;
- Displaying the at least one pharmacokinetic parameter.

The luminescent agent is particularly injected into the sample or the sample is treated with the luminescent agent. Other methods to provide and target the luminescent agent to the sample are also possible. When the luminescent agent is provided to the sample according to these methods, the sample comprises said luminescent agent and is ready for the measurement of the luminescence emitted by the agent, particularly after an incubation period in the sample.

During the incubation period it is possible that the luminescent agent develops its luminescent property, binds to the targets in the sample, and/or is processed further. The agent is particularly injected in or otherwise provided to a subject, and a tissue sample is obtained from said subject. The obtained tissue sample comprising the luminescent agent is then used for the method according to the invention. Particularly, organs of the subject can be analyzed and a distribution of the agent in the various organs can be determined and compared to each other.

The term "tissue sample" particularly refers to organs; tissue or parts thereof. Particularly an organ on a chip is also comprised by the meaning of "tissue sample". A tissue sample in the meaning of the specification is particularly a tissue excretion. The term "body fluid" particularly refers to any fluid that can be produced by a body, such as blood, a liquid excretion, or other liquids of the body.

The term "non-mammalian animal" is particularly limited to animals where no professional medical expertise is required in order to carry out the necessary steps for the method according to the invention, particularly with respect to the administration of the agent. Such anon-mammalian animal is for example a zebrafish embryo.

The luminescent agent can be for example fluorescent or phosphorescent. The agent can comprise a plurality of particularly different fluorophores. The agent can be configured to report certain environmental properties for example by means of a change in emission properties. Such a change can be based for example on a quenching process like Forster-Resonance-Energy Transfer or an emission wavelength shift of the emitted photons.

Furthermore, the agent can comprise a compound to be tested, such as for example a liposome or a drug. For this purpose, a luminescent portion can be attached to the compound forming the luminescent agent. The compound can be a drug, with specific properties that are to be verified or falsified in the sample.

The photons can be recorded by appropriate detectors, wherein particularly each detection channel comprises a separate detector of the fluorescence measurement system.

A correlation function is particularly calculated or computed with a computer from the recorded photons. The correlation function is particularly determined from the intensity of the recorded photons, wherein the intensity is a number of photons per time.

Of particular interest are the fluctuations of the recorded intensity, as fluorescence correlation spectroscopy evaluates the fluctuation in the recorded intensity. The signal contributing to the temporal behavior of the correlation function are fluctuations caused by the luminescent agent entering and exiting the detection volume of the measurement system. Uncorrelated noise does not contribute to the temporal behavior of the correlation function, but leads to an offset of the correlation function.

The correlation function is particularly a second order autocorrelation function that correlates a recorded signal, i.e. the recorded photons, with itself or a second order cross-correlation function that correlates two different signals for a given lag time or a plurality of lag times.

The correlation function can be calculated for a single or a plurality of lag times. In the meaning of the present specification, a correlation function can also be a higher-order correlation function or even a higher-order cumulant function of the recorded signal.

The correlation function can be plotted, particularly with the lag times plotted logarithmically along the horizontal axis and the value of the correlation function for the specific time lag(s). Thus, the term correlation function is particularly used synonymously to the plot of the correlation function in the context of the specification. In order to be able to establish a temporal behavior of the characteristic parameter in the sample, the recorded photons are particularly processed in portions, wherein each portion comprises the recorded photons during a time interval of the measurement. These intervals can be overlapping or subsequent to each other. For each portion the correlation function is calculated and further evaluated in terms of the characteristic parameter such that changes of the characteristic parameter over time can be observed.

The fluorescence measurement setup is configured to record emitted photons at a signal to noise ratio that is sufficiently high for calculating the correlation function. The fluorescence measurement setup is particularly a confocal measurement system comprising a confocal measurement volume such as a confocal fluorescence microscope.

The confocal measurement volume from which emitted photons from the agent are collected and recorded is usually in the range of femtolitres.

It is noted that the correlation function can be calculated according and corresponding to various fluorescence correlation techniques and methods. Besides calculating the correlation function conventionally, a preprocessing of the recorded photons can be performed prior to the calculation of the correlation function. According to an embodiment of the invention, the recorded photons are acquired and processed with a fluorescence lifetime FCS method (FLCS), using a pulsed light source, with Fluorescence Cross-Correlation Spectroscopy (FCCS), total internal reflection (TIR)-FCS, with dual-focus (or two-focus) FCS (2fFCS), having two laterally shifted excitation volumes and pulsed interleaved excitation of these voumes, and/or scanning fluorescence correlation spectroscopy.

All these methods allow determine the characteristic parameter based on at least one correlation function.

In order to gain a full overview of the sample it is possible to record the emitted photons at different locations of the sample.

For each location the at least one pharmacokinetic parameter can be determined. The characteristic parameter is for example a parameter that is suited to reduce the information comprised in the correlation function to a single value or more values that is/are characteristic for the specific correlation function recorded from the sample. From the plurality of recorded and calculated correlation functions the plurality of the at least one characteristic parameter is determined such that a temporal course of the at least one characteristic parameter is recorded. The temporal course is associated with the temporal dynamic of the recorded photons from the agent. Therefore, changes in the at least one characteristic parameter can be monitored over time. From these changes, drug kinetics and other information can be extracted. The determination can for example be performed by fitting an appropriate diffusion model comprising the at least one characteristic parameter as a fit parameter to the calculated correlation function.

From the temporal course of the characteristic parameter a pharmacokinetic parameter is determined. The pharmacokinetic parameter reflects properties of the agent and the sample and can be used for further assessment. In order to be able to assess the pharmacokinetic parameter the at least one pharmacokinetic parameter is displayed for example on a display or another suitable device and/or is particularly digitally stored on a digital storage device.

Processing of the recorded photons is particularly facilitated by digitally storing the records of the photons, i.e. the signal, on a computer.

With the method according to the invention the drawbacks of radioactively labeled agents are overcome.

Even minor concentrations of the agent in the tissue sample can be detected, particularly because fluorescence correlation spectroscopy is a measurement method for detecting low concentrations of an analyte down to the picomolar range. While fluorescence correlation spectroscopy (FCS) has been tested for a qualitative assessment of test compounds, quantitative assessments with regard to a pharmacokinetic parameter have not been established.

According to another embodiment of the invention, the luminescent agent is a fluorescent agent, wherein the photons are fluorescence photons emitted by the fluorescent agent.

A fluorescent agent is particularly an auto-fluorescent agent or moiety of a component.

According to another embodiment of the invention, the sample consists or comprises of a body fluid, a tissue sample obtained from a subject, such as a rodent or a person, a non-mammalian animal or a cell culture.

According to another embodiment of invention, the characteristic parameter is at least one of:
- a photon count rate;
- a photon count rate per moiety comprising the luminescent agent, i.e. a molecular brightness;
- a concentration of the moiety comprising the luminescent agent; and/or a diffusion time of the moiety comprising the luminescent agent.

For example, the sample can be a tissue sample and the characteristic parameter that is determined for each correlation function is the count rate. This embodiment resembles counting of radioactive decays known in the state of the art and can therefore be processed analogously.

Alternatively, the sample can be a body fluid and the characteristic parameter can be a concentration and/or a count rate.

Furthermore, for example, the sample can be a body fluid and the characteristic parameter can be the concentration and/or the diffusion time.

All other possible combinations of samples, i.e. body fluid, tissue sample or non-mammalian animal and characteristic parameters are herewith also explicitly included.

Also, a combination of the characteristic parameters according to this embodiment can be used for the analysis of the temporal course.

The evaluation of the count rate from the correlation function is particularly advantageous as an average count rate can be derived with high fidelity from a correlation function (or the plotted curve corresponding to the correlation function). Apart from this, also a molecular brightness, i.e. a count rate per luminescent moiety (for example comprising a plurality of luminescent agents) can be derived from the correlation function straight forwardly.

It is possible to account for an uncorrelated background signal and to infer the count rate of the luminescent agent or the moiety comprising the luminescent agent(s) with higher accuracy from the correlation function than by directly measuring the count rate, i.e. intensity at the detector(s).

According to another embodiment of the invention, the excitation is a pulsed excitation such that a luminescence, particularly fluorescence lifetime can be determined from the luminescent agent. The fluorescence lifetime can be evaluated together with or separately from the correlation function.

According to another embodiment of the invention, the at least one pharmacokinetic parameter is a temporal decay value characterizing ,e.g. by means of fitting a model function to the temporal course of the characteristic parameter, particularly wherein a dose of the luminescent agent that is comprised by the sample is provided, particularly wherein the agent has been injected into the sample or the sample has been treated with the agent, and particularly wherein a clearance and/or a volume of distribution is determined from the temporal decay value.

A temporal decay value is for example a half-life time such as the T50 value that indicates when a signal has decayed to 50% of its initial value.

The temporal decay can have a plurality of decay times or just a single decay time, depending on the underlying processes.

The analysis of the temporal course of the characteristic values and the determination of the at least one pharmacokinetic parameter reduces the information gathered by the recorded photons to a single or only a few parameters that are of pharmacological interest. These parameters are commonly referred to as pharmacokinetic parameters.

The term "clearance" refers to its medical meaning in the context of the specification, particularly indicating the process of removing the agent from specific portions of the tissue or organ.

The term "volume of distribution" also refers to the medical and biomedical meaning in the context of the specification. The volume of distribution is also known as apparent volume of distribution.

The term "area under the curve" refers to its medical meaning in the context of the specification, particularly to the meaning as used in the field of pharmacokinetics.

According to this embodiment the pharmacokinetic parameter that is determined is the temporal decay value characterizing, e.g. by means of fitting a model function to the temporal course of the characteristic parameters. From such a decay value it is possible for example to estimate washout rates or amounts that remain in the sample. This information in turn can be used to estimate a pharmacological or toxicological effect the agent or the drug might have on the sample.

Moreover, it might be useful to know the initial dose of the luminescent agent that has been present in the sample when the experiment started. With this additional information it is possible to calculate the clearance and/or a volume of distribution. According to another embodiment of the invention, the at least one pharmacokinetic parameter is a volume of distribution.

The volume of distribution is particularly interesting when the sample is a body fluid such as blood.

According to another embodiment of the invention, the at least one pharmacokinetic parameter is an area under the curve of the temporal course of the characteristic parameter.

The area under the curve is frequently estimated in radioactively labeled experiments and thus the results achieved can be translated and compared to previously conducted experiments with radioactively labeled substances.

According to another embodiment of the invention, the area under the curve is determined from the concentration of the moiety comprising the luminescent agent. According to another embodiment of the invention, the characteristic parameters determined from the correlation functions are histogramed and particularly displayed by a display device.

From the histograms other kinetics or dynamics of the sample can be derived. According to another embodiment of the invention, the sample is blood or an organ homogenisate.

These body fluids are particularly well-suited for estimating drug dynamics and kinetics.

Furthermore, the sample can be one of amniotic fluid; aqueous humour; vitreous humour; bile; blood plasma; blood serum; breast milk; cerebrospinal fluid; cerumen (earwax); chyle; chime; endolymph and perilymph; exudates; feces - diarrhea; female ejaculate; gastric acid; gastric juice; lymph; mucus (including nasal drainage and phlegm); pericardial fluid; peritoneal fluid; pleural fluid; pus; rheum; saliva; sebum (skin oil); serous fluid; semen; smegma; sputum; synovial fluid; sweat; tears; urine; vaginal secretion; vomit.

According to another embodiment of the invention, the characteristic parameters and the pharmacokinetic parameter are determined for a plurality of samples, wherein the samples are different organs or parts thereof of the same subject, particularly wherein the characteristic parameters for each organ are histogramed.

This allows for direct comparison of the agent distribution in the organs and thus provides a better understanding of the distribution of the agent in the body. According to another embodiment of the invention, the sample is a zebrafish or a zebrafish embryo.

Zebrafish is a well-established model organism for pharmacological studies.

The agent can be injected into the zebrafish or the zebrafish embryo and the agent dynamics can be monitored with the method according to the invention in the living zebrafish. For this purpose, the zebrafish can be immobilized.

According to another embodiment of the invention, the sample is an organ on a chip. The organ on a chip can be evaluated while the agent is processed by the organ on a chip.

The organ on a chip is considered a tissue sample in the context of the specification. According to another embodiment of the invention, the sample is a body fluid, wherein the body fluid is provided by a catheter that drains the body fluid from a body of a subject, wherein the sample is excited and the photons are recorded from a location outside of the body and wherein the sample is introduced back into the body of the subject particularly after the sample has been excited and the photons recorded.

For this reason, the catheter can comprise a measurement portion that is suited to perform the excitation and collection of photons of the sample in a manner that the signal to noise ratio and the optics are sufficiently good for performing fluorescence correlation spectroscopy.

The catheter particularly comprises or is connected to a measurement chamber or a microfluidic chip in the measurement portion in the region where the agent is excited and the photons are recorded from.

Alternatively the catheter comprises a transparent tubing portion for the excitation and collection of photons.

This embodiment allows for a direct determination of the pharmacokinetic parameter on a living subject.

In case the body fluid flows (in contrast to standing still) through the catheter, the correlation functions can be evaluated taking into account or simply estimating said flow.

According to another embodiment of the invention, the sample is excited with a pulsed light and wherein the correlation functions are calculated with a fluorescence lifetime correlation analysis, wherein the analysis is configured to generate correlation functions that are corrected for non-fluorescent and/or luminescent background contributions.

Fluorescence lifetime correlation analysis is for example taught by [1].

This embodiment allows for a more accurate estimation of the count rate by filtering out contributions that are not related to the luminescence of the luminescence agent, but are for example caused by afterplusing of the detectors, scattering or fluorescent moieties that fluoresce in the same spectral region than the luminescent agent but are of no interest. These moieties can be filtered out by their fluorescence lifetime that is intentionally different to the fluorescence lifetime of the agent.

Moreover, different states of the agent can be separated (e.g. bound/unbound) by applying the fluorescence lifetime correlation analysis.

The characteristic values can then be specifically determined for the correct and desired signal, which yields a more accurate determination of the pharmacokinetic parameter.

According to another embodiment of the invention, the temporal decay value comprises or is a half-life period and/or a decay time.

The problem is also solved by a computer program.

The computer program according to the invention comprises computer program code, wherein when the computer program is executed on a computer, the computer program executes the method according to one of the preceding claims.

The term "computer" refers to a state of the art common computer particularly equipped with appropriate computer programs and an operating system for executing at least the computer-implementable steps, such as the calculation of the correlation functions, the determination of the characteristic parameter and the pharmacokinetic parameter.

In the following the invention is detailed with exemplary embodiments. It is shown in
- Fig. 1: an embodiment of the invention, wherein the sample is a zebrafish;
- Fig. 2A: various histograms of the characteristic parameters;
- Fig. 2B: various temporal courses of the characteristic parameters estimated from the correlation functions for various luminescent agents in a zebrafish sample.
- Fig. 3A: histograms of the characteristic parameters estimated for different organs of a rat.
- Fig. 3B: fluorescent and radioactive count rate determined for each organ of a rat;
- Fig. 4: a catheter comprising blood for recording measurement data; and
- Fig. 5: a schematic flow diagram, illustrating the method according to the invention.

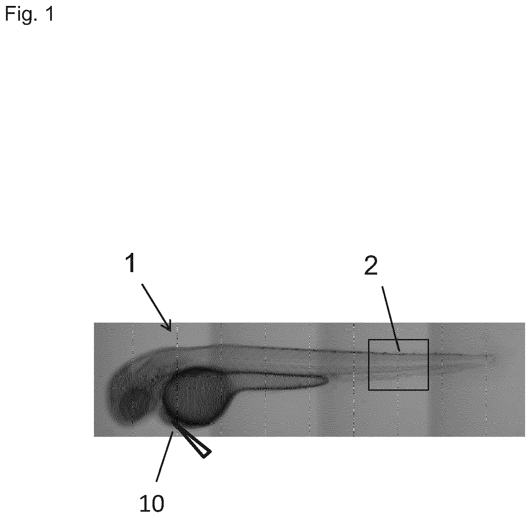

In Fig. 1 it is schematically shown how the method according to the invention is performed *in vivo,* when the sample 1 is a zebrafish embryo. The luminescent agent 2, here a nanoparticle formulation comprising for example micelles, quantum dots, liposomes and/or polymersomes, is injected via micro-injection 10 in the zebrafish. The injection 10 is not a surgical step and can for example be performed by laboratory staff. In general, independent of the biological sample 1, the agent 2 can be also coupled to a compound such as a drug to be tested. The agent, particularly together with the compound, is then processed by the zebrafish.

At the tail portion of the zebrafish, the sample is excited 300 with a confocal fluorescence microscope in order to record photons emitted from the agent. The recorded photons are then processed by a computer that executes a computer 6 program for determining the fluorescence correlation function, particularly the autocorrelation function of the recorded photons, wherein the recorded photon stream is processed piecewise, such that a plurality of fluorescence correlation functions is generated. For example, the recorded photon stream can be subdivided in portions of one minute duration. Every individual portion is then processed and a fluorescence correlation function is calculated. For each correlation function characteristic parameters of the correlation function are calculated. This is achieved for example by fitting an appropriate model to the calculated correlation function. The characteristic parameters can be a diffusion time, a count rate, the concentration and a molecular brightness.

Then, the characteristic parameters can be histogramed and also be plotted versus time. Particularly from the plot, pharmacokinetic parameters including area under the curve or half-life can be determined.

The area under the curve is a pharmacokinetic parameter. Of particular interest is the area under the curve of the count rate.

### Detailed example:

In order to determine the area under the curve with respect to the count rate the following steps are executed:
   (a) Calibration of the fluorescence measurement system by adjusting the confocal volume, the laser wavelength for excitation and detector fit assets according to the fluorescence properties of the tested compound in solution.
   (b) Performing a test measurement in solution on the compound labelled with the agent and calculating a correlation function from the recorded photons. From this correlation function and in particular from the measured diffusion time the confocal volume of the fluorescence measurement system can be estimated by referencing the measured diffusion time against a provided or calculated diffusion coefficient of the compound in solution.
   (c) Immobilizing a zebrafish embryo or larvae that are particularly younger than 144 hours post fertilisation. The immobilisation of the embryos can be achieved by embedding the zebrafish in agarose, e.g. 0.3% w/v, comprising a local anaesthetic such as 0.16 mg/mL tricaine and if needed phenylthiourea, e.g. 0.03 mg/mL to prevent pigmentation for imaging.
   (d) Injecting a defined volume and concentration of the compound labelled with the fluorescent agent into the blood circulation for example via the Duct of Cuvier or the caudal vein into the zebrafish embryo using a pneumatic pico pump.
   (e) Positioning the injected zebrafish with the appropriate portion into the confocal volume.
   (f) Exciting and recording the fluorescence from the labelled compound.
   (g) Calculating an autocorrelation function from the recorded fluorescence signal for a predefined measurement interval such as to generate a plurality of autocorrelation functions that are determined from different temporal portions of the recorded fluorescence signal.
   (h) Determining the characteristic parameter count rate and molecular brightness, i.e. counts per molecule from each autocorrelation function by means of a mathematical fit algorithm. The fit algorithm is particularly configured to model a non-compartmental or compartmental diffusion.
   (i) Normalizing the count rates to time zero, i.e. the measurement start time and plot the normalized count rate versus time.
   (j) Determining pharmacokinetic parameters such as plasma/tissue half-life, area under the curve, mean residence time, clearance, volume of distribution of the injected test compounds.

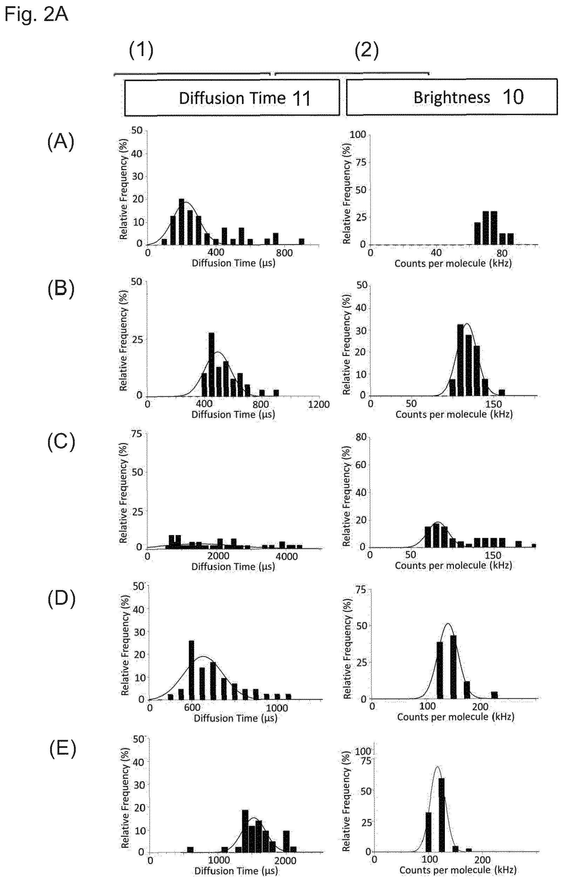

In Fig. 2A results are shown for various liposomes fluorescently labeled using Dil, i.e. a lipophilic indocarbocyanine dye which is incorporated in the lipid bilayer membrane. The liposomes were injected in zebrafish as described above. The measurement was performed in the dorsal artery of the zebrafish embryo.

The following liposome compositions were tested. Row (A) DSPC based liposomes comprising 10% cholesterol; row (B) DSPC based liposomes comprising 50% cholesterol; row (C) liposomes comprising DOPC; row (D) DSPC-cholesterol based liposomes comprising PEG 2000; row (E) DSPC-cholesterol based liposomes comprising PEG 5000. The experiment was performed over 4 hours and correlation functions have been calculated every 10 minutes, each time point for a total period of 1 minute. The characteristic parameters diffusion time (column (1)) and molecular brightness (column (2)) were determined and histogramed for the first 30 min.

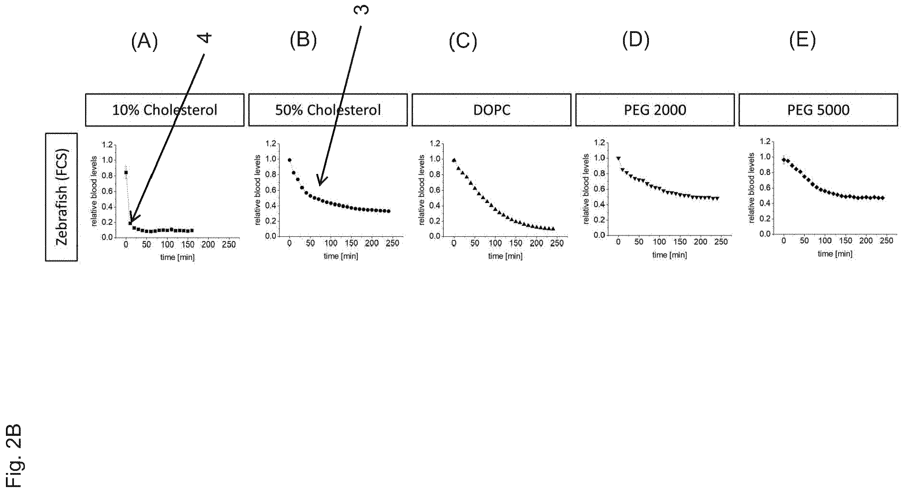

In Fig. 2B the results for the characteristic parameter plotted versus time are shown for the same experiment as in Fig. 2A.

On the vertical axis (y-axis) the normalized count rate is plotted and on the horizontal axis (x-axis) the time is plotted.

On the left column (column (A)) the liposome comprises 10% cholesterol, in column (B) the liposome comprises 50% cholesterol, in column (C) the liposome comprises DOPC, in column (D) the liposome comprises PEG 2000 and in column (E) the liposome comprises PEG 5000.

It can be seen that the blood circulation half-life increases from column (A) to column (E). These results were confirmed by corresponding experiments with radioactive markers in rodents.

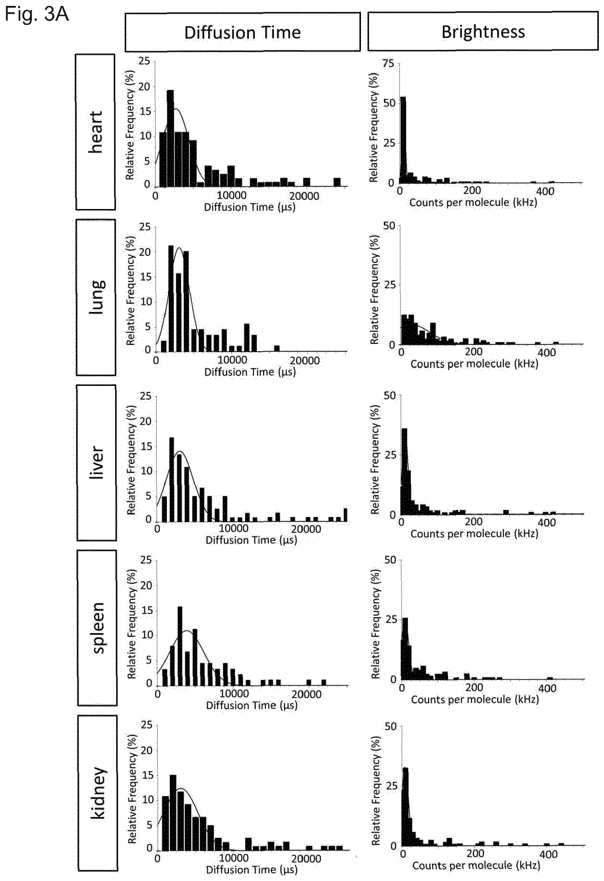

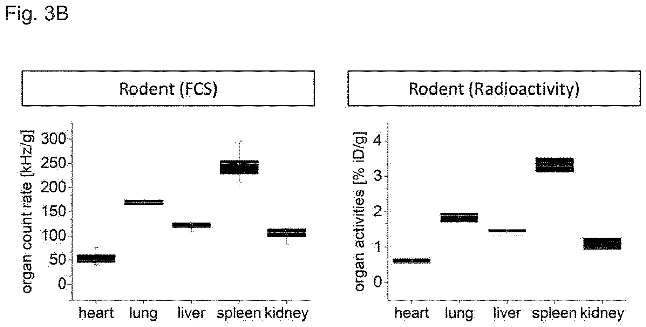

In Fig. 3A and Fig. 3B the tested samples were organs from a rat. The rats had been injected with PEG 2000 liposomes labeled with a fluorescent agent and a radioactive tracer. The organs were harvested after 4 hours. The method according to the invention was performed on the harvested organs.

The characteristic parameters diffusion time and molecular brightness were determined from the plurality of auto-correlation functions for each organ and histogramed (Fig. 3A). Furthermore, in Fig. 3B the characteristic parameter count rate was evaluated for each organ as well as the radioactive decay counts from each organ has been determined.

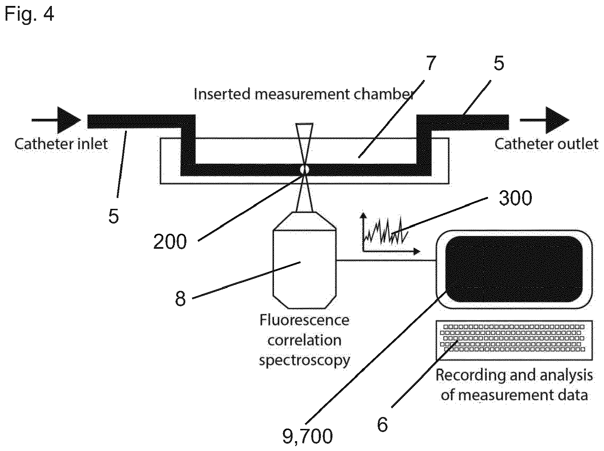

In Fig. 4 an embodiment of the invention is shown where the sample is a body fluid, namely blood and wherein the body fluid is provided to a measurement chamber 7 via a catheter 5. A spectroscope with an objective lens 8 focusses the laser light and detects the emitted photons. In the measurement chamber 7 the sample 1 is excited 200 and the emitted photons are recorded 300 for correlation analysis. Afterwards, the body fluid is drained from the measurement chamber and can be reinserted (not shown) to the body of the subject.

The recorded photons are processed according to the invention and the at least one pharmacokinetic parameter is displayed 700 on a display 9 of a computer 6.

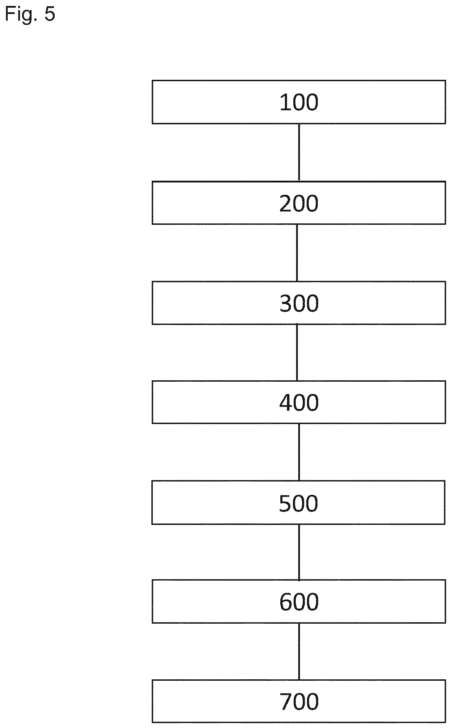

In Fig. 5 a schematic flow diagram of the method according to the invention is shown. Each rectangle indicates a method step, wherein the respective number in the rectangle indicates which method step is performed. First a biological sample 1 is provided 100 with a luminescent agent. The biological sample 1 more specifically the luminescent agent 2 is excited 100 with an appropriate excitation light. The emitted photons are recorded 300 by an appropriate photon detector. Form the recorded photon a plurality of correlation functions is calculated 400. Subsequently for each correlation function at least one characteristic parameter is determined 500 from which a temporal course 3 is determined. From the temporal course 3 of the at least one characteristic parameter 4 at least one pharmacokinetic parameter is determined. This pharmacokinetic parameter is displayed 700 on a display.

### References

[1] Gosh et al., "Fluorescence lifetime correlation spectroscopy: Basics and applications", Methods, Volumes 140-141, 1 May 2018, Pages 32-39, https://doi.org/10.1016/j.ymeth.2018.02.009

## Claims

1. A fluorescence correlation spectroscopy method for determination of a pharmacokinetic parameter in a biological sample comprising at least the steps of:
- Providing (100) a biological sample (1) comprising a luminescent agent (2);
- Exciting (200) the sample with light such that the luminescent agent is excited and emits photons;
- Recording (300) the emitted photons;
- Calculating (400)a plurality of correlation functions from the recorded photons, wherein the correlation functions are calculated for temporally subsequent or overlapping record portions comprising a plurality of consecutively recorded photons;
- Determining (500) for each correlation function at least one characteristic parameter (4) from the correlation function and determining a temporal course (3) of the at least one characteristic parameter (4);
- From the temporal course (3) of the at least one estimated characteristic (4) parameter determining (600) at least one pharmacokinetic parameter;
- Displaying (700) the at least one pharmacokinetic parameter.

2. The method according to claim 1, wherein the biological sample (1) comprises or consists of a body fluid, a tissue sample obtained from a subject, a non-mammalian animal, or a cell culture.

3. The method according to claim 1 or 2, wherein the characteristic parameter is at least one of:
- a photon count rate;
- a photon count rate (10) per moiety comprising the luminescent agent;
- a concentration of the moiety comprising the luminescent agent; and/or
- a diffusion time (11) of the moiety comprising the luminescent agent.

4. The method according to one of the preceding claims, wherein the at least one pharmacokinetic parameter is a temporal decay value of the temporal course of the characteristic parameter (4), particularly wherein a dose of the agent that is comprised by the biological sample (1) is provided, and wherein a clearance and/or a volume of distribution is determined from the temporal decay value.

5. The method according to one of the preceding claims, wherein the at least one pharmacokinetic parameter is a volume of distribution.

6. The method according to one of the preceding claims, wherein the at least one pharmacokinetic parameter is an area under the curve of the temporal course of the characteristic parameter.

7. The method according to claim 6, wherein the area under the curve is estimated for the concentration of the moiety comprising the luminescent agent.

8. The method according to one of the preceding claims, wherein the characteristic parameters (4) are histogramed.

9. The method according to one of the preceding claims, wherein the biological sample (1) is blood or an organ homogenisate.

10. The method according to one of the preceding claims, wherein the characteristic parameters (4) and the pharmacokinetic parameter are determined for a plurality of samples (1), wherein each sample is a different organ or a part thereof of the same subject or obtained from the same subject, particularly wherein the characteristic parameters for each organ are histogramed.

11. The method according to one of the preceding claims, wherein the biological sample (1) is an organ on a chip.

12. The method according to one of the preceding claims, wherein the sample is a body fluid, wherein the body fluid is provided by a catheter (5) that drains the body fluid from a body of a subject, wherein the biological sample (1) is excited (200) and the photons are recorded (300) outside of the body and wherein the biological sample (1) is introduced back into the body of the subject after the biological sample (1) has been excited (200) and the photons recorded (300).

13. The method according to one of the preceding claims, wherein the biological sample (1) is excited with a pulsed light and wherein the correlation functions are calculated by a fluorescence lifetime correlation analysis, wherein the analysis is configured to generate correlation functions that are corrected for non-fluorescent and/or luminescent background contributions.

14. The method according to one of the preceding claims, wherein the temporal decay value comprises or is a half-life period and/or a decay time.

15. Computer program comprising computer program code which, when run on a computer (6), executes the method according to one of the preceding claims.
